# EUROPEAN PATENT SPECIFICATION

(11) **EP 0 457 456 B1**
(45) Date of publication and mention of the grant of the patent: **27.12.1996**
(21) Application number: 91303901.2
(22) Date of filing: 30.04.1991
(51) Int. Cl.: A61M 25/00

(54) **Multiple layer high strength balloon for dilatation catheter**
Mehrschichtiger hochfester Ballon für Dilatationskatheter
Ballonnet multicouche à haute résistance pour cathéter dilatateur

(30) Priority: 15.05.1990 US 523490
(43) Date of publication of application: 21.11.1991
(73) Proprietor: C.R. BARD, INC., Murray Hill New Jersey 07974 (US)
(72) Inventor: Saab, Mark A., Lawrence, Massachusetts 01841 (US)
(74) Representative: Woodward, John Calvin

(56) References cited:
- EP-A- 0 357 562
- FR-A- 2 328 482
- US-A- 3 539 674
- US-A- 4 327 736

## Description

This invention relates to balloons used in dilatation catheters and to methods for making such balloons.

Balloon dilatation catheters are used in the treatment of a variety of vascular conditions. Among the more frequent uses for balloon dilatation catheters is in vascular angioplasty of the peripheral and coronary arteries, by which arteries obstructed by plaque (formed by fatty deposits such as cholesterol) are dilated to improve blood flow through the artery. In a typical angioplasty procedure, a balloon dilatation catheter is inserted percutaneously into the patient's arterial system and then is advanced and steered through the patient's arteries until the distal end of the catheter, that carries the balloon, is disposed adjacent the obstruction (stenosis). The balloon end of the catheter then is advanced into the stenosis and, when so placed, is inflated under high pressure, to dilate the artery in the region of stenosis. The catheter typically is used with a small diameter steerable guidewire which is used to guide the catheter to the stenosis. By way of example, such a catheter and guidewire system is disclosed in US Patent 4,545,390.

It is desirable, particularly in coronary angioplasty in which the coronary arteries are narrow and tortuous, and in which the stenoses often may be calcified and difficult to dilate, that the catheter and its balloon meet a number of stringent requirements. Among these are that the balloon be capable of folding down to a low profile about the catheter shaft so that the balloon portion of the catheter is more readily insertable through the stenosis. Inability to insert the balloon portion of the catheter into the stenosis is among the more frequent causes of an unsuccessful angioplasty. Also among the important characteristics of the balloon dilatation catheter is that it should be "trackable", that is, it must be able to follow and advance over the guidewire and through the artery even when the artery is highly tortuous with many sharp bends. An additional important characteristic of the balloon is that it should have a high burst strength so that it may dilate hard, calcified stenoses as well as those that require less force for the dilatation.

In order to improve the low profile and trackability characteristics of the character in the region of the balloon, efforts have been made to develop dilatation balloons having very thin walls so that the balloon will fold more readily to a low profile about the catheter shaft and also so that the balloon will be more flexible, thus enhancing the ability of the catheter to bend in the region of the balloon, thereby achieving improved trackability. To that end, significant advances have been made in the art. US Patent 4,490,421 describes the manufacture of dilatation balloons by which balloons may be made having a high burst strength and significantly thinner walls than its predecessors. The procedure was improved further, as described in US patent application serial No. 001,759 filed January 9, 1987 (corresponding document: EP-A-274411, published Aug. 13, 1988) to enable the manufacture of high strength balloons having even thinner, more flexible walls.

French 2,328,482, which is used as a basis for the preamble of claim 1, discloses a balloon for a dilatation catheter, the interior of the balloon being in fluid communication with an inflation lumen within the dilatation catheter when mounted on said dilatation catheter, the balloon being formed from polymeric material and having a cylindrical midportion, an outwardly tapering conical portion at each end of the midportion and a cylindrical neck portion at the ends of the conical portions.

Although the foregoing advances in manufacturing thinner walled balloons have significantly improved the catheters, those efforts have been directed at the cylindrical midportion of the balloon. The cones and necks of the balloon, at the ends of the cylindrical midportion, are not as thin as the cylindrical midportion. Each cone is of increasing wall thickness in a direction away from the cylindrical midportion of the balloon and reaches a maximum wall thickness at its juncture with the necks. The wall thickness of the neck is at that maximum value throughout their length. The increased wall thickness of the balloon in the regions of the cones and the necks detracts from the ability of the balloon to collapse to a low profile as well as the ability of the balloon to track along the guidewire along sharp tortuous paths. It would be desirable, therefore, to provide a balloon for a dilatation catheter in which the wall thickness in the cone and neck portions is reduced and, preferably, is not substantially greater than the thickness in the cylindrical midportion of the balloon. It is among the objects of the invention to provide such a balloon and method for its manufacture.

The present invention provides an improvement over the prior art in that the balloon is formed from at least two layers including an inner and outer layer, the outer layer terminating intermediate the length of the conical portions of the inner layer to provide a stepped configuration to reduce the wall thickness in the cone and neck portions.

The invention further provides a method for making a dilatation balloon having a cylindrical midportion, cones extending from the ends of the midportions and necks extending from the ends of the cones, the balloon being formed from a polymeric material comprising at least two layers and comprising the steps specified in claim 6.

The balloon of the present invention is formed from at least two thin layers rather than from a single unitary layer, the aggregate wall thickness of the layers being approximately equal to the wall thickness of a conventional single layer balloon in the cylindrical midportion. More specifically, the balloons are made by the following steps: a) blow moulding a balloon in a cylindrical mould from a thin walled polymeric tubular parison; b) the balloon is then removed from the mould and is trimmed at its ends to remove the necks and a portion of the cones; c) the trimmed balloon then is replaced in the mould against the mould walls.

A second polymeric tube then is inserted into the mould and it is blow moulded, expanding outwardly against the confines of the cylindrical mould and the inner surface of the trimmed balloon; d) the assembly of balloon layers is then removed from the mould and the last formed layer is trimmed at its end to define the neck portion of the balloon. If the balloon is to be formed with more than two layers, steps b) and c) are repeated until the required number of layers has been formed, the subsequent layers in step b) being trimmed to be slightly longer than the previous trimmed balloon and to form a stepped configuration in the cone. The resulting balloon thus formed has cones in which the wall thickness does not increase substantially and in which the wall thickness in the neck region also is substantially less than with prior techniques for making such balloons. The resulting balloon is therefore more flexible in the cone and neck region than prior balloons.

The balloon dilatation catheter of the invention also has improved low profile and trackable characteristics in the region of its cones and necks.

The cone and neck regions of the dilatation balloon of the invention are not substantially greater in wall thickness than the cylindrical midportion of the balloon, the balloon being formed from a plurality of thin layers in intimate contact with each other.

Embodiments of the invention will now be described, by way of example only, with reference to the accompanying drawings in which:
FIGURE 1 is an illustration of a balloon dilatation catheter;
FIGURE 2 is an enlarged diagrammatic cross-sectional illustration of a conventional balloon in which the thicknesses of the balloon material are highly exaggerated to illustrate the relative thicknesses of the balloon at the central cylindrical portion, the cone portion and the neck portion;
FIGURE 3 is an enlarged diagrammatic cross-sectional illustration of a balloon made in accordance with the present invention;
FIGURE 4 is an illustration of the mould process used in making the balloon; and
FIGURES 5A-5E are diagrammatic illustrations of the mould and the sequence of steps for making the balloon of the present invention.

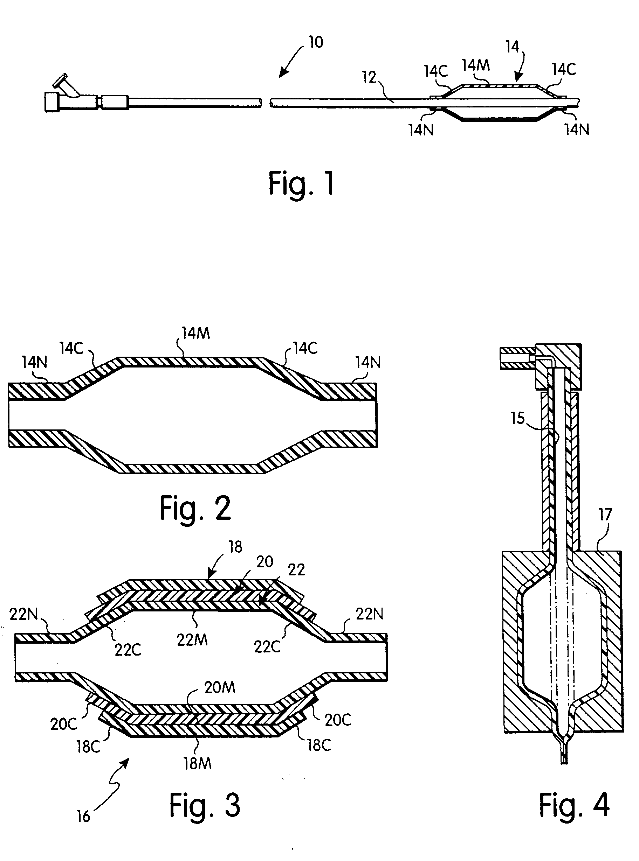

Figure 1 illustrates a balloon dilatation catheter of the type with which the present invention is concerned. The catheter 10 has a proximal end (to the left in Figure 1) and a distal end (to the right in Figure 1). An elongate flexible shaft 12 typically is provided with appropriate lumens, for example, a guidewire lumen (not shown) that extends the length of the shaft and an inflation lumen (not shown) that extends from the proximal end of the shaft to the distal region of the shaft and communicates with the interior of a dilatation balloon 14 that is mounted to the distal region of the shaft. By way of example, the catheter shaft 12 may be of the order of 150cm long. The balloon may vary in size from about 1.5mm to 4.5mm diameter, for coronary use. The balloon may be considered as having a constant diameter cylindrical midportion 14M which expands to the nominal diameter, a pair of end cones 14C at the ends of the midsection 14M and a pair of neck sections 14N that extend outwardly from the narrow ends of the cones 14C. The balloon 14 is attached to the catheter shaft 12 by adhesively bonding the necks 14N to the catheter shaft 12.

Figure 2 shows a conventional balloon formed in one piece, shown with its wall thicknesses exaggerated for ease of illustration. Such a balloon may be made according to the procedure described in US patent 4,490,421 and US application serial No. 001,759 (EP-A-274411). The balloon is formed in a biaxially stretching process that includes blow moulding in a mould of the type illustrated in Figure 4. As described in further detail in US patent 4,490,421 a tubular parison 15 of uniform inner and outer diameters and wall thickness is extended through the mould 17. The tubular parison is stretched axially and is blow moulded radially within the mould 17. The portion of the tube 15 that forms the cylindrical midportion 14M is subjected to a greater degree of radial stretching than the neck portions 14N. Consequently, the midportion 14M will have less wall thickness than the neck portion 14N. The cones 14C are radially stretched to progressively varying degree as the diameter of the cones change. Thus, as illustrated in Figure 2 the midportion 14M will have the thinnest wall, the neck 14N will have the thickest wall and the cones 14C will have a progressively increasing wall thickness in a direction extending from the ends of the midportion 14M to the necks 14N. The cones 14C and necks 14N thus are thicker than required. The increased thickness in the cones adversely affects the ability of the balloon to contract to a low profile. The greater thickness of the cones and the necks detracts from the trackability of the catheter.

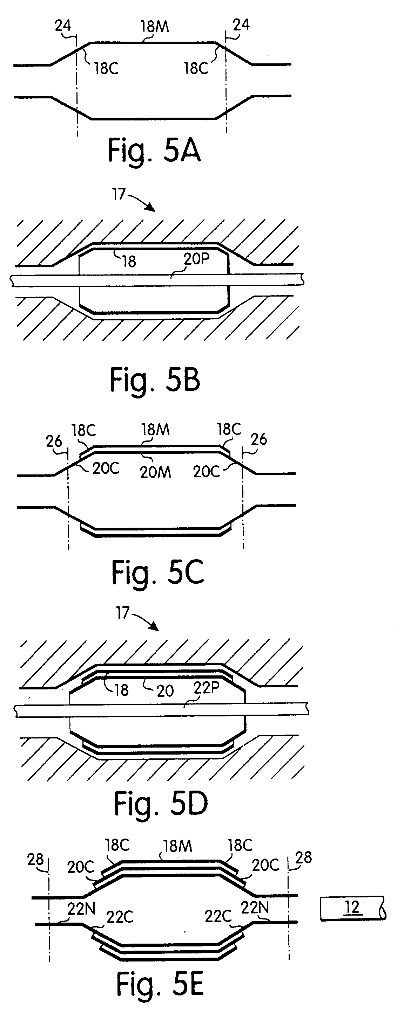

In accordance with the present invention, the balloon is formed from a plurality of relatively thin layers rather than a single unitary relatively thick layer. The configuration of a balloon made in accordance with the invention is shown in Figure 3 in high diagrammatic enlarged and exaggerated form. The illustrated balloon, indicated generally at 16, is formed from three layers including an outer layer 18, an intermediate layer 20 and an inner layer 22. The layers 18,20,22 are each in continuous intimate contact with each other and need not be adhesively attached. The balloon 16 is formed in a procedure illustrated diagrammatically in Figures 5A-5E. The mould 17, described in further detail in US patent 4,490,421 receives a tubular parison 15 of the polymer from which the balloon is to be formed. The parison is relatively thin walled. The parison is stretched and expanded biaxially by combined axial stretching and blow moulding as described in US patent 4,490,421, to form a balloon having cones and neck extensions. In accordance with the invention, a first such balloon 18 is formed and is heat set at an elevated temperature as described in said US application serial No. 001,759 (EP-A-274411) and is then removed from the mould. The first balloon then is trimmed at each end between the ends of its cones as suggested at 24 in Figure 5A, thus leaving a balloon midportion 18M and a pair of partial cones 18C. The first balloon, thus trimmed, then is replaced in the mould. A second elongate tubular parison 20P then is inserted into the mould as suggested in Figure 5B and it is biaxially stretched and expanded. When the second parison 20P expands, it contacts fully and intimately the inner surface of the outer layer 18 contained within the mould. The second balloon thus formed also is heat set. After the intermediate balloon 20 has been formed, the combined outer and intermediate balloons 18,20 are removed from the mould.

The ends of the intermediate balloon layer 20 then are trimmed as suggested at 26 in Figure 5C so that the intermediate cones 20C extend slightly outwardly of the shorter outer cones 18C. The two layer, partially formed balloon then may be reinserted into the mould and the process repeated with a third parison 22P of polymeric material as suggested in Figure 5D. When the third layer 22 has been formed, the assembly of layers is again removed from the mould. The ends of the inner layer 22 then may be trimmed as suggested at 28 in Figure 5E to leave the necks 22N and an exposed portion of the cones 22C. The balloon thus formed may be attached to the catheter shaft 12 by adhesively bonding the necks 22N to the catheter shaft.

It will be appreciated from the foregoing that the neck of the multiluminal balloon which is formed from an initial thin parison, although not expanded as much as the balloon midportion still is substantially thinner than the corresponding neck in a balloon formed in one piece in a single layer. The regions of the cones similarly define a series of stepped thicknesses in which the thickness of the cone decreases in a direction extending away from the balloon midportion. Thus, although the cone segment in each of the three layers will tend to have increased thickness in an outward direction, the stepped down configuration of the cones, considered together, results overall in a cone thickness that is relatively small. For example, even in a thin wall high strength balloon made in accordance with the procedure described in US application serial No. 001,759 (EP-A-274411), the wall thickness in the cone ranges from about 0.008mm (0.0003") at its juncture with the cylindrical midportion to approximately 0.0254mm (0.001") at its juncture with the neck. The neck portion may be of the order of 0.0254mm (0.001").

By way of example, balloons made in accordance with the present invention may be formed from a tubular parison of polyethylene terephthalate having an inner diameter of the order of 0.426mm (0.0168") inner diameter and a wall thickness of the order of 0.056mm (0.0022"). The parison is biaxially stretched about 3X in an axial direction and radially about 7X inner diameter stretch and about 5.5X outer diameter stretch. The resulting balloon will have a wall thickness in the cylindrical midportion region of the order 0.0025mm (0.0001"), a cone thickness gradually increases from 0.0025mm (0.0001") to about 0.0101mm (0.0004"). The aggregate wall thickness in the cylindrical midportion of the multiple layers is of the order of 0.008mm (0.0003") which is comparable to currently commercially available balloons.

From the foregoing, it will be appreciated that the invention provides a new construction for a dilatation balloon, a new method for its manufacture and a new resulting catheter that will display improved characteristics as to trackability and reduced profile.

## Claims

1. A balloon for a dilatation catheter, the interior of the balloon being in fluid communication with an inflation lumen within the dilatation catheter when mounted on said dilatation catheter, the balloon (16) being formed from polymeric material and having a cylindrical midportion (14M), an outwardly tapering conical portion (14C) at each end of the midportion and a cylindrical neck portion (14N) at the ends of the conical portions, **characterised in that** the balloon (16) is formed from at least two layers including an inner and outer layer (22,18), the outer layer (18) terminating intermediate the length of the conical portions (22C) of the inner layer (22), to provide a stepped configuration to reduce the wall thickness in the cone and neck portions.

2. A balloon as claimed in claim 1 characterised by at least three layers (18,20,22), at least one intermediate layer (20) being disposed between the inner layer (22) and the outer layer (18), and the intermediate layer (20) being of a length between that of the inner and outer layers (22,18).

3. A balloon as claimed in claims 1 or 2 characterised in that the layers are in intimate adhesive-free contact with each other.

4. A balloon as claimed in claims 1 or 2 characterised in that the more inwardly disposed layers are of greater length than the more outwardly disposed layers.

5. A balloon as claimed in any preceding claim when mounted on an elongate flexible shaft (12) to provide dilatation catheter (10).

6. A method for making a dilatation balloon (16) having a cylindrical midportion (14M), cones (14C) extending from the ends of the midportion (14M) and necks (14N) extending from the ends of the cones (14C), the balloon (16) being formed from a polymeric material comprising at least two layers and comprising the steps of:
a) blow moulding a balloon having cones and neck extensions in a mould (17);
b) removing the balloon from the mould (17) and trimming its ends to remove the neck extensions and a portion of the cones;
c) placing the trimmed balloon in the mould (17) and then blow moulding a subsequent balloon layer into intimate contact with the previously placed balloon segment;
d) in the case where the balloon is formed of more than two layers, repeating steps b) and c) until the required number of layers has been formed, whereby in step b) the subsequent layers are trimmed to be slightly longer than the previous trimmed balloon and to form a stepped configuration in the cone; and
e) removing the assembly of balloon layers (18,20,22) from the mould and trimming the last formed layer (22) to define the neck portion (22N) of the balloon (16).

7. A method for making a dilatation balloon as defined in claim 6 wherein the balloons (16) are stretched axially during their formation whereby the balloon layers (18,20,22) are biaxially oriented.

8. A method for making a dilatation balloon as defined in claims 6 or 7 further comprising heat setting each balloon layer (18,20,22) after its formation at a temperature elevated above the temperature at which the balloon layers are blow moulded.

## Patentansprüche

1. Ballon für einen Dilatationskatheter, wobei das Innere des Ballons in Fluidverbindung mit einem Aufblashohlraum in dem Dilatationskatheder steht, wenn er an dem Dilatationskatheder angebracht ist, wobei der Ballon (16) aus Polymermaterial besteht und einen zylindrischen Mittelabschnitt (14M), einen sich nach außen verjüngenden, konischen Abschnitt (14C) an jedem Ende des Mittelabschnitts und einen zylindrischen Halsabschnitt (14N) an den Enden der konischen Abschnitte aufweist, **dadurch gekennzeichnet**, daß der Ballon (16) aus wenigstens zwei Schichten einschließlich einer inneren und einer äußeren Schicht (22,18) besteht, wobei die äußere Schicht (18) in der Mitte der Länge der konischen Abschnitte (22C) der inneren Schicht (22) endet, so daß eine abgesetzte Form entsteht und die Wanddicke in den konischen und den Halsabschnitten verringert wird.

2. Ballon nach Anspruch 1, **gekennzeichnet durch** wenigstens drei schichten (18,20,22), wobei wenigstens eine Mittelschicht (20) zwischen der inneren Schicht (22) und der äußeren Schicht (18) angeordnet ist, und die Mittelschicht (20) eine Länge aufweist, die zwischen der der inneren und der der äußeren Schicht (22,18) liegt.

3. Ballon nach Anspruch 1 oder 2, **dadurch gekennzeichnet**, daß die schichten in engem, klebstofffreiem Kontakt miteinander sind.

4. Ballon nach Anspruch 1 oder 2, **dadurch gekennzeichnet**, daß die mehr innen angeordneten Schichten eine größere Länge aufweisen als die mehr außen angeordneten Schichten.

5. Ballon nach einem der vorangehenden Ansprüche, wenn er an einem länglichen flexiblen Schaft (12) angebracht ist, so daß ein Dilatationskatheder (10) entsteht.

6. Verfahren zum Herstellen eines Dilatationsballons (16) mit einem zylindrischen Mittelabschnitt (14M), konischen Abschnitten (14C), die sich von den Enden des Mittelabschnitts (14M) aus erstrecken, sowie Halsabschnitten (14N), die sich von den Enden der konischen Abschnitte (14C) aus erstrecken, wobei der Ballon (16) aus einem Polymermaterial besteht und wenigstens zwei schichten umfaßt, das die folgenden Schritte umfaßt:
a) Blasformen eines Ballons mit konischen Abschnitten und Halsverlängerungen in einer Form (17);
b) Entnehmen des Ballons aus der Form (17) und Beschneiden seiner Enden, um die Halsverlängerungen und einen Teil der konischen Abschnitte zu entfernen;
c) Einlegen des beschnittenen Ballons in die Form (17) und anschließendes Blasformen einer folgenden Ballonschicht, die in engem Kontakt mit dem zuvor eingelegten Ballonteil ist;
d) wenn der Ballon aus mehr als zwei Schichten besteht, Wiederholen der Schritte b) und c), bis die erforderliche Anzahl von schichten hergestellt worden ist, wobei in Schritt b) die anschließenden Schichten so beschnitten werden, daß sie etwas größer sind als der zuvor beschnittene Ballon, so daß eine abgesetzte Form in dem konischen Abschnitt entsteht; und
e) Entnehmen der Einheit aus Ballonschichten (18,20,22) aus der Form und Beschneiden der zuletzt hergestellten Schicht (22), um den Halsabschnitt (22N) des Ballons (16) zu bilden.

7. Verfahren zum Herstellen eines Dilatationsballons nach Anspruch 6, wobei die Ballons (16) bei ihrer Herstellung axial gestreckt werden, so daß die Ballonschichten (18,20,22) biaxial ausgerichtet sind.

8. Verfahren zum Herstellen eines Dilatationsballons nach Anspruch 6 oder 7, das des weiteren das Heißfixieren jeder Ballonschicht (18,20,22) nach ihrer Herstellung bei einer Temperatur umfaßt, die über der Temperatur liegt, bei der die Ballonschichten blasgeformt werden.

## Revendications

1. Ballonnet pour cathéter de dilatation, l'intérieur du ballonnet étant en communication fluidique avec un orifice de gonflage à l'intérieur du cathéter de dilatation lorsqu'il est monté sur ledit cathéter de dilatation, le ballonnet (16) étant formé à partir d'un matériau polymère et ayant une partie médiane cylindrique (14M), une partie conique (14C) s'amincissant vers l'extérieur à chaque extrémité de la partie médiane et une partie de col cylindrique (14N) aux extrémités des parties coniques, caractérisé en ce que le ballonnet (16) est formé à partir d'au moins deux couches comprenant une couche intérieure et extérieure (22, 18), la couche extérieure (18) se terminant à mi-longueur des parties coniques (22C) de la couche intérieure (22), pour fournir une configuration en gradins pour réduire l'épaisseur de paroi dans les parties coniques et de col.

2. Ballonnet selon la revendication 1, caractérisé par au moins trois couches (18, 20, 22), au moins une couche intermédiaire (20) étant disposée entre la couche intérieure (22) et la couche extérieure (18), et la couche intermédiaire (20) étant d'une longueur comprise entre celle des couches intérieure et extérieure (22, 18).

3. Ballonnet selon les revendications 1 ou 2, caractérisé en ce que les couches sont en contact étroit sans adhérence les unes avec les autres.

4. Ballonnet selon les revendications 1 ou 2, caractérisé en ce que les couches disposées les plus à l'intérieur sont de longueur plus grande que les couches disposées les plus à l'extérieur.

5. Ballonnet selon l'une quelconque des revendications précédentes lorsqu'il est monté sur un axe flexible allongé (12) pour fournir un cathéter de dilatation (10).

6. Procédé pour fabriquer un ballonnet de dilatation (16) ayant une partie médiane cylindrique (14M), des cônes (14C) s'étendant depuis les extrémités de la partie médiane (14M) et des cols (14N) s'étendant des extrémités des cônes (14C), le ballonnet (16) étant formé à partir d'un matériau polymère comprenant au moins deux couches et comprenant les étapes consistant à :
a) souffler un ballonnet ayant des cônes et des extensions formant col dans un moule (17) ;
b) retirer le ballonnet du moule (17) et à ébarber ses extrémités pour retirer les extensions du col et une partie des cônes ;
c) placer le ballonnet ébarbé dans le moule (17) et ensuite souffler une couche de ballonnet ultérieure en contact étroit avec le segment de ballonnet placé précédemment ;
d) dans le cas où le ballonnet est formé de plus de deux couches, répéter les étapes b) et c) jusqu'à ce que le nombre requis de couches ait été formé, moyennant quoi dans l'étape b) les couches ultérieures sont ébarbées pour être légèrement plus longues que le ballonnet ébarbé précédent et pour former une configuration en gradins dans le cône ; et
e) retirer l'assemblage de couches de ballonnet (18, 20, 22) du moule et ébarber la dernière couche formée (22) pour définir la partie de col (22N) du ballonnet (16).

7. Procédé pour fabriquer un ballonnet de dilatation selon la revendication 6, dans lequel les ballonnets (16) sont étirés axialement pendant leur formation, moyennant quoi les couches de ballonnet (18, 20, 22) sont orientées biaxialement.

8. Procédé pour fabriquer un ballonnet de dilatation selon les revendications 6 ou 7, comprenant en outre le durcissement de chaque couche de ballonnet (18, 20, 22) après sa formation à une température située au-dessus de la température à laquelle les couches de ballonnet sont soufflées.
